# EUROPÄISCHE PATENTSCHRIFT

(11) **EP 0 579 576 B1**
(45) Veröffentlichungstag und Bekanntmachung des Hinweises auf die Patenterteilung: **20.05.1998**
(21) Anmeldenummer: 93810481.7
(22) Anmeldetag: 06.07.1993
(51) Int. Cl.: C07C 39/06, C08K 5/13, C09K 15/08

(54) **2,4-Dialkyl-6-sec-alkylphenole**
2,4-Dialkyl-6-sec-alkylphenols
2,4-Dialkyl-6-sec-alkylphénols

(30) Priorität: 15.07.1992 EP 92810538
(43) Veröffentlichungstag der Anmeldung: 19.01.1994
(73) Patentinhaber: Ciba Specialty Chemicals Holding Inc., 4057 Basel (CH)
(72) Erfinder: Dubs, Paul, Dr., CH-1723 Marly (CH); Pitteloud, Rita, Dr., CH-1724 Praroman (CH)

(56) Entgegenhaltungen:
- EP-A- 0 406 169
- GB-A- 1 396 107
- US-A- 3 183 273
- PATENT ABSTRACTS OF JAPAN, vol. 12, no. 286 (M-727)(3133), 5. August 1988; & JP-A-63 062 781
- PATENT ABSTRACTS OF JAPAN, vol. 2, no. 34 (C-77)(4519), 8. März 1978; & JP-A-52 136 261

## Beschreibung

Die vorliegende Erfindung betrifft neue 2,4-Dialkyl-6-*sec*-alkylphenole und damit gegen thermischen, oxidativen und aktinischen Abbau stabilisiertes organisches Material.

Eine Reihe von Trialkylphenolen, z. B. 2,6-Di-*tert*-butyl-4-methylphenol (®Swanox BHT), und deren Verwendung zum Stabilisieren von organischem Material sind bekannt. In US-A-3 511 802 wird die Stabilisierung von Polypropylenharzen mit alkylsubstituierten Phenolen, z.B. 2,6-Bis(1-methylheptyl)-p-cresol, offenbart. In Chemical Abstracts 106:32349u wird die Verwendung von sterisch gehinderten Phenolen, wie z. B. 2-*sec*-Butyl-4,6-di-*tert*-butylphenol, in Stabilisatormischungen, welche zum Abtrennen von Nebenprodukten bei der Herstellung von 2-(2-Chlorethoxy)ethanol verwendet werden, beschrieben. Dem Derwent Abstract 75900Y/43 ist die Verwendung von sterisch gehinderten Phenolen, beispielsweise 2,6-Diisopropyl-4-octadecylphenol, als Stabilisatoren für Vinylchloridharze zu entnehmen. In der Britischen Patentanmeldung Nr. 1 396 107 werden 2,6-Diisopropyl-4-t-alkyl-phenole als Edukte zur Herstellung von 2,6-Diisopropylphenol verwendet. In US-A-5 098 945 sind 2,4-Dimethyl-6-s-alkylphenole als Stabilisatoren offenbart.

Gegenstand der Erfindung sind Verbindungen der Formel I 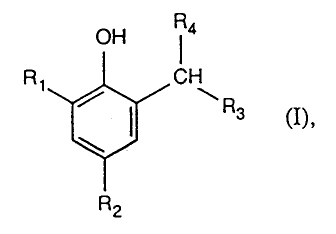 worin
- R₁: C₁-C₄-n-Alkyl bedeutet,
- R₂: C₄-C₁₈-tert-Alkyl oder α,α-Dimethylbenzyl ist,
- R₃: für C₁-C₂₈-Alkyl steht und
- R₄: Methyl oder Ethyl bedeutet,
mit der Massgabe, dass die Gruppe -CHR₃R₄ mindestens 4 C-Atome enthält.

R₁ als C₁-C₄-n-Alkyl ist Methyl, Ethyl, n-Propyl, n-Butyl. R₁ ist bevorzugt Methyl oder Ethyl, insbesondere Methyl.

Unter R₂ als C₄-C₁₈-tert-Alkyl ist ein Rest -CXYZ zu verstehen, wobei X, Y und Z unabhängig voneinander C₁-C₁₅-Alkyl bedeuten und die Summe der Zahl der Kohlenstoffatome in allen 3 Alkyleinheiten (X+Y+Z) 3 bis 17 ist R₂ bedeutet also beispielsweise t-Butyl, 2-methyl-but-2-yl, 2-methyl-pent-2-yl, 2-methyl-hept-2-yl, 2-methyl-non-2-yl, 2-methyl-undec-2-yl, 2-methyl-heptadec-2-yl, 3-methyl-pent-3-yl, 3-methyl-hept-3-yl, 3-methyl-non-3-yl, 3-methyl-undec-3-yl, 3-methyl-heptadec-3-yl, usw.
Bevorzugt ist R₂ t-Butyl.

R₃ als C₁-C₂₈-Alkyl kann linear oder verzweigt sein und ist z.B. Methyl, Ethyl, Propyl, Butyl, Pentyl, Hexyl, Heptyl, Octyl, Nonyl, Decyl, Dodecyl, Octadecyl, Icosyl, Docosyl, Pentacosyl, Hexacosyl oder Octacosyl.
Insbesondere ist R₃ C₁-C₂₂-Alkyl, z.B. C₈-C₂₂-Alkyl, insbesondere C₁₀-C₁₈-Alkyl, vorzugsweise C₁₂-C₁₈-Alkyl.
Bevorzugt ist R₃ lineares Alkyl.

R₄ ist Methyl oder Ethyl, vorzugsweise Methyl.

Die Gruppe -CHR₃R₄ enthält 4-31, z.B. 6-31, insbesondere 10-25, vorzugsweise 13-21 Kohlenstoffatome.

In bevorzugten Verbindungen der Formel I ist R₁ Methyl oder Ethyl.

Weiterhin bevorzugt sind Verbindungen der Formel I, worin R₁ Methyl ist, R₂ C₄-C₈-tert-Alkyl bedeutet und R₃ C₁-C₂₂-n-Alkyl ist.

Besonders bevorzugt sind Verbindungen der Formel I, worin R₁ Methyl bedeutet.

Andere bevorzugte Verbindungen der Formel I sind solche, worin R₄ Methyl ist.

In anderen bevorzugten Verbindungen der Formel I ist R₃ C₈-C₂₂-n-Alkyl.

In weiteren interessanten Verbindungen der Formel I bedeutet R₂ C₄-C₈-tert-Alkyl, insbesondere tert-Butyl.

Beispiele für Verbindungen der Formel I sind:
4-t-Butyl-2(1-methyl-undecyl)-6-methylphenol,
4-t-Butyl-2(1-methyl-tridecyl)-6-methylphenol,
4-t-Butyl-2(1-methyl-pentadecyl)-6-methylphenol,
4-t-Butyl-2(1-methyl-heptadecyl)-6-methylphenol,
6-Methyl-4(1,1,3,3-tetramethylbutyl)-2(1-methyl-undecyl)-phenol,
6-Methyl-4(1,1,3,3-tetramethylbutyl)-2(1-methyl-tridecyl)-phenol,
6-Methyl-4(1,1,3,3-tetramethylbutyl)-2(1-methyl-pentadecyl)-phenol,
6-Methyl-4(1,1,3,3-tetramethylbutyl)-2(1-methyl-heptadecyl)-phenol,
4(α,α-Dimethylbenzyl)-2(1-methyl-undecyl)-6-methylphenol,
4(α,α-Dimethylbenzyl)-2(1-methyl-tridecyl)-6-methylphenol,
4(α,α-Dimethylbenzyl)-2(1-methyl-pentadecyl)-6-methylphenol,
4(α,α-Dimethylbenzyl)-2(1-methyl-heptadecyl)-6-methylphenol.

Die Verbindungen der Formel I können in Analogie zu bekannten Verfahren hergestellt werden, beispielsweise durch katalytische Orthoalkylierung von 2,4-Dialkylphenolen mit α-Olefinen: 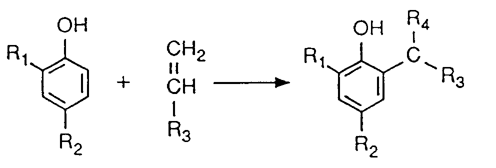 R₁, R₂, R₃ und R₄ sind wie in Anspruch 1 definiert. Dieses Verfahren kann z.B. in Analogie zu den in US-A-3 766 276 beschriebenen Methoden durchgeführt werden.

Die Umsetzung wird zweckmässigerweise bei Temperaturen von 80-250°C, bevorzugt 180-230°C, in Gegenwart eines Katalysators durchgeführt. Geeignete Katalysatoren sind beispielsweise Aluminiumphenolate, wobei zweckmässigerweise das Aluminiumphenolat des entsprechenden verwendeten Phenols eingesetzt wird. Es können auch Aluminiummetall, Aluminiumoxid, Aluminiumalkoholate oder Trialkylaluminium verwendet werden, wobei dann während der Reaktion das entsprechende Aluminiumphenolat gebildet wird. In der Regel wird der Katalysator in einer Menge von 1-10 Mol% zugesetzt.

Die Verbindungen der Formel I können auch hergestellt werden, indem in ein 2-substituiertes Phenol zunächst durch Orthoalkylierung der -CR₃R₄ Rest eingeführt wird und danach durch anschliessende Paraalkylierung der Substituent R₂.
Als geeignete Katalysatoren für den Orthoalkylierungsschritt bei der Anwendung dieser Methode seien genannt:
a) anorganische und organische Säuren, wie z.B. Schwefelsäure oder p-Toluolsulfonsäure;
b) Zeolithe, z.B. ZSM-Zeolith;
c) saure Erden, z.B ®Fulmont 234, ®Fulcat 14 oder ®Fulmont 700;
d) Friedel-Crafts-Katalysatoren, wie z.B in Kozlikovski Ya. B. et al., Zh. Org. Khim. 23, 1918-24 (1987); Laan J.A.M.; Chem. Ind. 1, 34-35 (1987) und Kurashev M.V. et al.; Izv. Akad. Nauk. SSSR, Ser. Khim. 8, 1843-1846 (1986) beschrieben;
e) aktives γ-Aluminiumoxid, wie z.B. in DE-B-1,142,873 und US-A-3,367,981 beschrieben.

Als Katalysator wird für diesen Reaktionsweg aktives γ-Aluminiumoxid besonders bevorzugt.

Der zweite Schritt, die Paraalkylierung wird danach mit in der Technik allgemein üblichen Methoden, wie z. B. Friedel-Crafts-Alkylierung, durchgeführt.

Bei der Umsetzung der Phenole mit α-Olefinen entstehen in der Hauptsache Verbindungen der Formel I, worin R₄ Methyl ist. Es ist aber auch möglich, dass Gemische von Verbindungen der Formel I, worin R₄ Methyl ist, mit solchen Verbindungen der Formel I, worin R₄ Ethyl ist, entstehen. Das Verhältnis der Verbindungen der Formel I mit R₄ = Methyl und denjenigen mit R₄ = Ethyl hängt hauptsächlich vom gewählten Herstellungsverfahren, sowie vom verwendeten Katalysator ab.
Wenn bei der Herstellung ein Gemisch aus den Verbindungen der Formel I anfällt, kann dieses z.B. mit Hilfe von chromatographischen Verfahren, insbesondere Gaschromatographie und Hochdruckflüssigchromatographie (HPLC), aufgetrennt werden. In der Regel ist eine Trennung von solchen Gemischen nicht erforderlich. Sie werden vorzugsweise direkt als Stabilisatoren für organisches Material eingesetzt, wie unten beschrieben.

Gegenstand der Erfindung sind auch Mischungen von Verbindungen der Formel I, worin R₄ Methyl bedeutet, mit solchen, worin R₄ Ethyl bedeutet.

Das Gewichtsverhältnis der Verbindungen der Formeln (I), worin R₄ Methyl bedeutet, mit solchen, worin R₄ Ethyl bedeutet ist z.B. 99:1 bis 1:99, bevorzugt 99:1 bis 90:10, insbesondere 95:5 bis 70:30.

Bevorzugt sind jene Mischungen, worin in den Verbindungen der Formel I mit R₄ = Methyl die Kettenlänge von R₃ um eins grösser ist als in den Verbindungen der Formel I mit R₄ = Ethyl.
Das sind die Mischungen, die direkt bei der Umsetzung der Phenole mit den α-Olefinen entstehen können. Dabei enthält der Rest R₃ zwangsläufig in den Verbindungen mit R₄ = Methyl eine Methylengruppe mehr als in den Verbindungen mit R₄ = Ethyl.

Die Phenole der Formel I und deren Mischungen eignen sich ausgezeichnet zum Stabilisieren von gegen thermischen, oxidativen oder aktinischen Abbau empfindlichem organischen Material. Besondere Wirksamkeit entfalten sie gegen die durch Sauerstoff und Wärme, insbesondere durch Sauerstoff, hervorgerufenen Abbauarten der genannten Materialien. Sie sind daher insbesondere als ausgezeichnete Antioxidantien einsetzbar.

Ebenfalls Gegenstand der Erfindung sind Zusammensetzungen, enthaltend (a) ein gegen oxidativen thermischen oder aktinischen Abbau empfindliches organisches Material und (b) mindestens eine Verbindung der Formel I, bzw. ein wie vorstehend beschriebenes Gemisch von Verbindungen.

Beispiele für solche organischen Materialien (a) sind:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder π- oder σ-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglykole, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, Polyisocyanaten oder Epoxidharzen vernetzt sind.
26. Vernetzte Epoxidharze, die sich von Polyepoxiden ableiten, z.B. von Bis-glycidylethern oder von cycloaliphatischen Diepoxiden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM; Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.
29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.
30. Wässrige Emulsionen natürlicher oder synthetischer Kautschuke, wie z.B. Naturkautschuk-Latex oder Latices von carboxylierten Styrol-Butadien-Copolymeren.

Bevorzugt sind Zusammensetzungen, worin die Komponente (a) ein Polystyrol, substituiertes Polystyrol, Co- oder Terpolymeres von Polystyrol oder substituiertem Polystyrol ist. Beispiele dafür sind oben insbesondere unter den Punkten 4., 5. und 6. genannt.

Besonders bevorzugt als Komponente (a) sind schlagfestes Polystyrol (IPS), Styrol-Acrylnitril-Copolymere (SAN) und Acrylnitril-Butadien-Styrol-Terpolymere (ABS), insbesondere Acrylnitril-Butadien-Styrol-Terpolymere (ABS) und Methylmethacrylat-Butadien-Styrol-Pfropfcopolymere (MBS).

Von Interesse sind als Komponente (a) auch Polycarbonat, Polyestercarbonat, Polyurethan, Polyamid, Copolyamid, Polyacetal und Polyphenylenoxid. Entsprechende Beispiele sind oben unter den Punkten 12., 14. und 15. zu finden.

Bevorzugt sind auch Zusammensetzungen, worin die Komponente (a) ein Polyolefin ist. Beispiele dafür sind insbesondere oben unter den Punkten 1.-3. angegeben, besonders interessant sind Polyethylen und Polypropylen.

Die erfindungsgemässen Zusammensetzungen enthalten zweckmässigerweise 0,01 bis 10 %, bevorzugt 0,05 bis 5 %, insbesondere 0,1 bis 2 % mindestens einer Verbindung der Formel I, oder einer Mischung von Verbindungen der Formel I, worin R₄ = Methyl ist mit solchen Verbindungen der Formel I, worin R₄ = Ethyl ist, bezogen auf das Gesamtgewicht des zu stabilisierenden organischen Materials.

Neben den Verbindungen der Formel I können die erfindungsgemässen Zusammensetzungen zusätzlich herkömmliche Additive enthalten, wie beispielsweise:
1. Antioxidantien
   1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
   1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.
   1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hhydroxyphenyl-stearat, Bis-(3,5-di-tert-butyl-4-hydroxyphenyl)adipat.
   1.4. Hydroxylierte Thiodiphenylether, z.B. 2,2 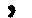 -Thio-bis-(6-tert-butyl-4-methylphenol), 2,2 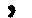 -Thio-bis-(4-octylphenol), 4,4 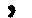 -Thio-bis-(6-tert-butyl-3-methylphenol), 4,4 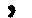 -Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.
   1.5. Alkyliden-Bisphenole, z.B. 2,2 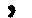 -Methylen-bis-(6-tert-butyl-4-methylphenol), 2,2 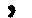 -Methylen-bis-(6-tert-butyl-4-ethylphenol), 2,2 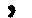 -Methylen-bis-[4-methyl-6-(α-methylcyclohexyl)-phenol], 2,2 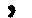 -Methylen-bis-(4-methyl-6-cyclohexylphenol), 2,2 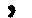 -Methylen-bis-(6-nonyl-4-methylphenol), 2,2 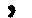 -Methylen-bis-(4,6-di-tert-butylphenol), 2,2 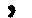 -Ethyliden-bis-(4,6-di-tert-butylphenol), 2,2 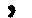 -Ethyliden-bis-(6-tert-butyl-4-isobutylphenol), 2,2 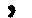 -Methylen-bis-[6-(α-methylbenzyl)-4-nonylphenol], 2,2 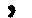 -Methylen-bis-[6-(α,α-dimethylbenzyl)-4-nonylphenol], 4,4 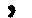 -Methylen-bis-(2,6-di-tert-butylphenol), 4,4 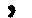 -Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3 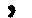 -tert-butyl-4 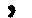 -hydroxyphenyl)-butyrat], Bis-(3-tert-butyl-4-hydroxy-5-methyl-phenyl)-dicyclopentadien, Bis-[2-(3 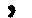 -tert-butyl-2 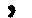 -hydroxy-5 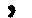 -methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.
   1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tert-butyl-4-hydroxybenzyl-mercaptoacetat.
   1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Didodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
   1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.
   1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.
   1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
   1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
   1.12. Ester der β-(3,5-Di-tert-butyl-4-hydroxyphenyl)-propionsäure mit ein- oder mehrwenigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N 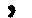 -Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.13. Ester der β-(5-tert-Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N 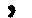 -Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.14. Ester der β-(3,5-Dicyclohexyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N 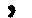 -Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.15. Ester der 3,5-Di-tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N 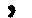 -Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
   1.16. Amide der β-(3,5-Di-tert-butyl-4-hydroxyphenyl)-propionsäure, wie z.B. N,N 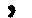 -Bis-(3,5-di-tert-butyl-4-hydroxyphenylpropionyl)-hexamethylendiamin, N,N 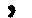 -Bis-(3,5-di-tert-butyl-4-hydroxyphenylpropionyl)-trimethylendiamin, N,N 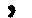 -Bis-(3,5-di-tert-butyl-4-hydroxyphenylpropionyl)-hydrazin.
2. UV-Absorber und Lichtschutzmittel
   2.1. 2-(2 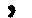 -Hydroxyphenyl)-benztriazole, wie z.B. 2-(2 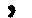 -Hydroxy-5 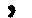 -methylphenyl)-benztriazol, 2-(3 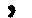 ,5 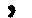 -Di-tert-butyl-2 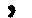 -hydroxyphenyl)-benztriazol, 2-(5 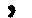 -tert-Butyl-2 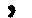 -hydroxyphenyl)-benztriazol, 2-(2 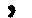 -Hydroxy-5 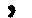 -(1,1,3,3-tetramethylbutyl)phenyl)-benztriazol, 2-(3 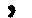 ,5 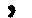 -Di-tert-butyl-2 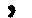 -hydroxyphenyl)-5-chlor-benztriazol, 2-(3 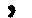 -tert-Butyl-2 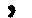 -hydroxy-5 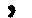 -methylphenyl)-5-chlor-benztriazol, 2-(3 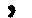 -sec-Butyl-5 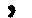 -tert-butyl-2 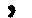 -hydroxyphenyl)-benztriazol, 2-(2 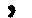 -Hydroxy-4 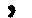 -octoxyphenyl)-benztriazol, 2-(3 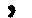 ,5 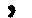 -Di-tert-amyl-2 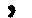 -hydroxyphenyl)-benztriazol, 2-(3 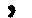 ,5 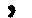 -Bis-(α,α-dimethylbenzyl)-2 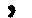 -hydroxyphenyl)benztriazol, Mischung aus 2-(3'-tert-Butyl-2 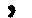 -hydroxy-5'-(2-octyloxycarbonylethyl)-phenyl)-5-chlor-benztriazol, 2-(3'-tert-Butyl-5'-[2-(2-ethylhexyloxy)-carbonylethyl]-2 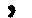 -hydroxyphenyl)-5-chlor-benztriazol, 2-(3'-tert-Butyl-2 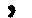 -hydroxy-5'-(2-methoxycarbonylethyl)phenyl)-5-chlor-benztriazol, 2-(3'-tert-Butyl-2 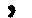 -hydroxy-5'-(2-methoxycarbonylethyl)phenyl)-benztriazol, 2-(3'-tert-Butyl-2 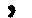 -hydroxy-5'-(2-octyloxycarbonylethyl)-phenyl)-benztriazol, 2-(3'-tert-Butyl-5'-[2-(2-ethylhexyloxy)carbonylethyl ]-2 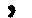 -hydroxyphenyl)-benztriazol, 2-(3'-Dodecyl-2 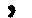 -hydroxy-5'-methylphenyl)-benztriazol, und 2-(3'-tert-Butyl-2'-hydroxy-5'-(2-isooctyloxycarbonylethyl)phenyl-benztriazol, 2,2'-Methylen-bis[4-(1,1,3,3-tetramethylbutyl)-6-benztriazol-2-yl-phenol]; Umesterungsprodukt von 2-[3'-tert-Butyl-5'-(2-methoxycarbonylethyl)-2'-hydroxy-phenyl]-benztriazol mit Polyethylenglycol 300; [R-CH₂CH₂-COO(CH₂)₃⁆₂ mit R = 3'-tert-Butyl-4'-hydroxy-5'-2H-benztriazol-2-yl-phenyl.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2 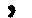 ,4 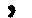 -Trihydroxy-, 2 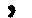 -Hydroxy-4,4 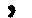 -dimethoxy-Derivat.
   2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
   2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2 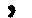 -Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure,- Kondensationsprodukt aus N,N 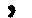 -Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl 4-piperidyl)-nitrilotriacetat, Tetrakis-(2,2,6,6-tetramethyl-4-piperidyl)-1,2,3,4-butantetraoat, 1,1 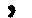 -(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.
   2.7. Oxalsäurediamide, wie z.B. 4,4 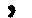 -Di-octyloxy-oxanilid, 2,2 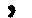 -Di-octyloxy-5,5 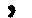 -di-tert-butyl-oxanilid, 2,2 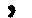 -Di-dodecyloxy-5,5 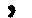 di-tert-butyl-oxanilid, 2-Ethoxy-2 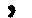 -ethyl-oxanilid, N,N 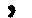 -Bis-(3-dimethylaminopropyl)-oxalamid, 2-Ethoxy-5-tert-butyl-2 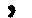 -ethyloxanilid und dessen Gemisch mit 2-Ethoxy-2 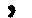 -ethyl-5,4 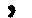 -di-tert-butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.
3. Metalldesaktivatoren, wie z.B. N,N 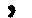 -Diphenyloxalsäurediamid, N-Salicylal-N 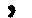 -salicyloylhydrazin, N,N 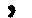 -Bis-(salicyloyl)-hydrazin, N,N 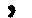 -Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Weitere Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4 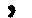 -biphenylen-phosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.
8. Nukleierungsmittel, wie z.B. 4-tert-Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.
10. Sonstige Zusätze, wie z.B. Weichmacher, Gleitmittel, Emulgatoren, Pigmente, Optische Aufheller, Flammschutzmittel, Antistatika, Treibmittel.
11. Benzofuranone bzw. Indolinone, wie z.B. in US-A-4 325 863 oder US-A-4 338 244 beschrieben.

Gegenstand der Erfindung ist weiterhin die Verwendung von Verbindungen der Formel I, bzw. der oben beschriebenen Mischungen von Verbindungen der Formel I zum

Stabilisieren von gegen thermischen, oxidativen oder aktinischen Abbau empfindlichem organischem Material.

Die Einarbeitung der Verbindungen der Formel I oder der Mischungen, sowie gegebenenfalls weiterer Additive in das organische Material erfolgt nach bekannten Methoden, beispielsweise vor oder während der Formgebung oder auch durch Aufbringen der gelösten oder dispergierten Verbindungen auf das organische Material, gegebenenfalls unter nachträglichem Verdunsten des Lösungsmittels. Die Verbindungen der Formel I können auch in Form eines Masterbatches, der diese beispielsweise in einer Konzentration von 2,5 bis 25 Gew.-% enthält, den zu stabilisierenden Materialien zugesetzt werden.

Die erfindungsgemässen Verbindungen können vor oder während der Polymerisation oder vor der Vernetzung zugegeben werden.

Die erfindungsgemässen Verbindungen können in reiner Form, als Lösungen, Dispersionen oder in Wachsen, Oelen oder Polymeren verkapselt in das zu stabilisierende Material eingearbeitet werden.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die erfindungsgemässen Verbindungen eignen sich auch als Kettenabbrecher bei der anionischen Lösungspolymerisation von 1,3-Dienen.

Die nachfolgenden Beispiele erläutern die Erfindung weiter. Angaben in Teilen oder Prozenten beziehen sich, ebenso wie in der übrigen Beschreibung und in den Patentansprüchen, auf das Gewicht, sofern nichts anderes angegeben ist.

### Beispiel 1:

### Herstellung von 4-tert-Butyl-2-(1-methyl-pentadecyl)-6-methylphenol

In einem Sulfierkolben von 2,5 l Inhalt wird eine Mischung aus 619 g (3,8 Mol) 4-tert-Butyl-2-methylphenol, 896 g (4 Mol) linearem α-Hexadecen und 38,5 g Aluminium-tris-(4-tert-butyl-2-methylphenolat) als Katalysator während 6 bis 8 Std. auf 215 °C erhitzt. Danach wird auf 70 °C abgekühlt, 30 ml 32 %ige Ammoniaklösung zugegeben und 30 Minuten bei 70 °C nachgerührt. Das ausgeschiedene Aluminiumhydroxid wird durch Filtration abgetrennt. Destillation des flüssigen Rückstandes im Hochvakuum (190-210 °C/0,001 mbar) ergibt 1,13 kg (77 % d. Theorie) 4-tert-Butyl-2-(1-methyl-pentadecyl)-6-methylphenol als farblose Flüssigkeit.

| Elementaranalyse: | | | | | |
|---|---|---|---|---|---|
| ber.: | C: | 83,44 % | gef.: | C: | 83,47 % |
| | H: | 12,45 % | | H: | 12,66 % |

### Beispiele 2-10:

Die Verbindungen der Beispiele 2-10 werden analog zu der Verbindung des Beispiels 1, unter Einsatz der entsprechenden Phenole und α-Olefine als Ausgangsprodukte, hergestellt. Ihre Strukturen und physikalischen Daten sind der Tabelle 1 zu entnehmen.

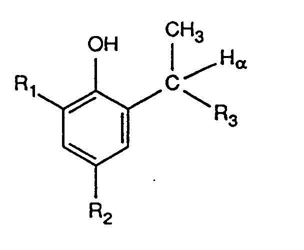

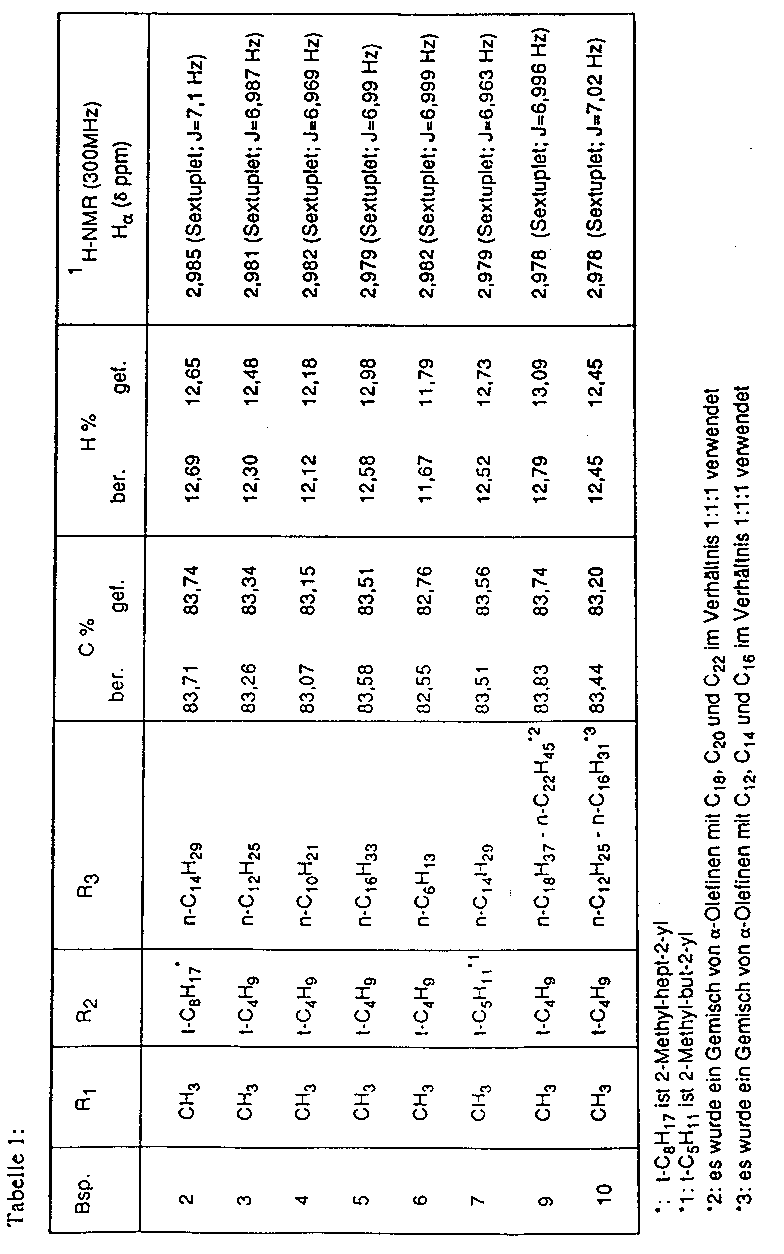

### Beispiel 11:

### Stabilisierung von Acrylnitril-Butadien-Styrol-Terpolymer (ABS)

Die in Tabelle 2 angegebenen Additive werden in 40 ml eines Lösemittelgemisches aus Hexan/Isopropanol gelöst. Die Lösung wird unter kräftigem Rühren zu einer Dispersion von 100 g ABS in 600 g Wasser gegeben, wobei die Lösung durch das ABS in kurzer Zeit (ca. einer Minute) vollständig absorbiert wird. Das ABS-Pulver wird abgenutscht und während 40 Stunden bei 40 °C im Vakuum getrocknet.Dem trockenen Pulver werden 2 % Titandioxid (Pigment), sowie 1 % Ethylen-bis-stearinsäureamid (Gleitmittel) zugegeben. Die Mischung wird anschliessend während 4 Minuten auf einem Zweiwalzenstuhl bei 180 °C compoundiert.
Aus dem Walzfell wird bei 175 °C eine Platte von 0,8 mm Dicke gepresst, aus welcher Prüflinge von 45 x 17 mm² ausgestanzt werden. Die Prüfung auf Wirksamkeit der zugesetzten Additive wird durch Hitzealterung in einem Umluftofen bei 180 °C vorgenommen. Als Kriterium dient die Farbentwicklung nach 45 Minuten Prüfdauer. Die Farbintensität wird mit dem "Yellowness Index" nach ASTM D 1925-70 bestimmt. Höhere Werte bedeuten intensivere Gelbfärbung. Die Versuche zeigen, dass die Gelbfärbung durch die zugesetzten erfindungsgemässen Verbindungen wirksam unterdrückt wird.

**Tabelle 2**

| Additiv | Yellowness Index nach 45 min bei 180 °C |
|---|---|
| - | 78 |
| 0,5 % DLTDP | 75 |

| 0,5 % DLTDP + 0,25 % der Verbindung aus Beispiel Nr. | Yellowness Index nach 45 min bei 180 °C |
|---|---|
| 1 | 30 |
| 2 | 30 |
| 3 | 30 |
| 4 | 30 |
| 5 | 28 |
| 6 | 30 |
| 7 | 29 |
| 9 | 30 |
| 10 | 31 |
| DLTDP = Dilaurylthiodipropionat | |

### Beispiel 12: Stabilisierung eines Polypropylens

100 Teile Polymer ®Statoil MF 4 (Polypropylen der Firma Petrokjemi Statoil, Norwegen; MF[230°C/2,16 kg] 4,3) werden mit 0,10 % des Phenols aus Beispiel 1 und 0,05 % Ca-Stearat in einem Brabender Plastographen bei 200 °C und 50 Umdrehungen pro Minute während 10 Minuten geknetet.
Das Prüfgemisch wird dann bei 200 °C während 6 Minuten zu 2 mm dicken Platten gepresst, und aus diesen Platten werden Scheiben mit 26 mm Durchmesser als Prüflinge ausgestanzt.
Von diesen Prüflingen wird der Yellowness Index bestimmt (Fotometer: Datacolor 3890, Blende 27, ohne UV, ohne Glanz, Normlicht C, 2° Detektor). Die Ergebnisse sind der Tabelle 3 zu entnehmen.

### Beispiel 13: Stabilisierung eines High Density Polyethylens

100 Teile ®Statoil H 870 (High Density Polyethylen der Firma Petrokjemi Statoil, Norwegen; MF[190°C/5,0 kg] 2,9) werden mit 0,10 % des Phenols aus Beispiel 1 und 0,05 % Ca-Stearat in einem Brabender Plastographen bei 200 °C und 50 Umdrehungen pro Minute während 10 Minuten geknetet.

Dann wird das Prüfgemisch bei 180 °C während 6 Minuten zu 2 mm dicken Platten gepresst, und aus diesen Platten werden Prüflinge in Form von Scheiben mit 26 mm Durchmesser ausgestanzt.
Von diesen Prüflingen wird der Yellowness Index bestimmt (Fotometer: Datacolor 3890, Blende 27, ohne UV, ohne Glanz, Normlicht C, 2° Detektor). Die Ergebnisse sind der Tabelle 3 zu entnehmen.

**Tabelle 3**

| Polymer aus | Yellowness Index |
|---|---|
| Beispiel 12 | 8,3 |
| Beispiel 13 | 8,2 |

### Beispiel 14: Stabilisierung eines Polypropylens

100 Teile ®Profax 6501 (Polypropylen der Firma Himont, USA; MF[230°C/2,16 kg] 3,2) werden mit 0,05 % des Phenols aus Beispiel 1 und 0,05 % Ca-Stearat bei max. 280 °C und 40 Umdrehungen pro Minuten mehrfach extrudiert.

Nach jedem Extrusionsvorgang wird ein Teil des Prüfgemisches entnommen und zu 2 mm dicken Platten gepresst. Die Pressbedingungen sind der Tabelle 4 zu entnehmen. Aus den gepressten Platten werden als Prüflinge zur Bestimmung des Yellowness Index (Fotometer: Datacolor 3890, Blende 27, ohne UV, ohne Glanz, Normlicht C, 2° Detektor) Scheiben mit 26 mm Durchmesser gestanzt. Die Ergebnisse sind in der Tabelle 4 aufgelistet.

**Tabelle 4**

| Pressbedingung °C/min | Yellowness Index nach Extrusion | | |
|---|---|---|---|
| | 1 | 3 | 5 |
| 260/6 | 3,5 | 3,9 | 4,3 |
| 300/6 | 3,4 | 3,6 | 4,2 |

### Beispiel 15: Stabilisierung von X-SBR Latex (Carboxylierter SBR Latex)

Jeweils 0,25 Gewichtsteile der in Tabelle 5 aufgeführten erfindungsgemässen Verbindungen werden in wenig Methanol gelöst und in 100 Gewichtsteile X-SBR-Latex (Styrol-Butadien-Copolymer) eingerührt. Anschliessend wird eine genau definierte Menge Latex in Petrischalen gefüllt und im Trockenschrank bei 80 °C getrocknet. Man erhält transparente Filme von ca. 0,2 mm Schichtdicke. Zu Vergleichszwecken wird eine Probe ohne Stabilisatoren hergestellt.
Die Prüfung auf Wirksamkeit der zugesetzten Stabilisatoren wird durch Hitzealterung in einem Umluftofen bei 150 °C vorgenommen. Nach den in Tabelle 5 angegebenen Zeitabständen wird die Verfärbung der Proben nach ASTM D 1925-70 (Yellowness INdex) bestimmt. Die Prüfergebnisse sind in Tabelle 4 zusammengestellt. Höhere Werte bedeuten intensivere Gelbfärbung.

**Tabelle 5**

| Stabilisator aus Beispiel | Yellowness-Index nach Alterung bei 150 °C nach | | |
|---|---|---|---|
| | 2 | 4 | 6 Stunden |
| - | 93 | * | * |
| 1 | 32 | 58 | 71 |
| 2 | 30 | 56 | 71 |
| 3 | 32 | 56 | 70 |
| 4 | 36 | 56 | 74 |
| 5 | 37 | 61 | 75 |
| 7 | 33 | 59 | 74 |
| 9 | 33 | 58 | 74 |
| 10 | 33 | 59 | 70 |

| | | | |
|---|---|---|---|
| * schwarz, nicht messbar | | | |

### Beispiel 16: Stabilisierung von Polyol

0,3 % des zu prüfenden Stabilisators werden in 20 g Lupranol®2045 (unstabilisiertes Polyol) so lange gerührt, bis eine klare, farblose Lösung erhalten wird. Jeweils 1000 mg dieser Lösungen werden in einem Abspaltungsgerät Rancimat® von Metrohm einem oxidationstest unterworfen.
Wenn über Polyol bei erhöhter Temperatur Sauerstoff geleitet wird, setzt eine Oxidationsreaktion ein. Die Reaktion lässt sich leicht nachweisen, da die gebildeten Oxidationsprodukte leicht flüchtig und zudem sauer sind, sodass sie nach Auffangen in Wasser die Leitfähigkeit (gemessen in µs/cm) der Lösung heraufsetzen. Durch Zusatz von Stabilisatoren wird diese Oxidationsreaktion quantitativ bis zum vollständigen Verbrauch des Stabilisators verhindert. Wird die Leitfähigkeit in Abhängigkeit von der Zeit gemessen, so ist der Beginn der Oxidation durch den Kurvenverlauf (lineare Steigung) zu erkennen. Die Zeit vom Beginn des Versuchs bis zum Kurvenanstieg ist ein Mass für die Wirksamkeit des Stabilisators. Die Ergebnisse sind der folgenden Tabelle 6 zu entnehmen.

**Tabelle 6**

| Stabilisator Verbindung aus Beispiel Nr. | Minuten bis zum Erreichen einer Leitfähigkeit von 25 µS/cm bei 150 °C in O₂ |
|---|---|
| Referenz * | 37 |
| 3 | 40 |
| 4 | 44 |
| 6 | 40 |

| | |
|---|---|
| * 2,6-di-t-butyl-p-cresol | |

### Beispiel 17: "Deposit and Oxidation Panel Test" (DOPT)

Beim "Deposit and Oxidation Panel Test" (DOPT) handelt es sich um eine Variante der Prüfmethode für Motorenöle, insbesondere für Dieselmotorenöle, welche durch G. Abellaneda et al IIIè Symposium CEC, 1989, 61, New Cavendish Street, London WIM8AR, England beschrieben wurde. Dabei wird die Eignung der Oele mit Stabilisator zur Verhinderung von Ablagerungen auf Kolben geprüft.
In einer feuchten Luft-Atmosphäre, die mit 260 ppm NO₂ und 26 ppm SO₂ angereichert ist, wird das zu prüfende Oel auf eine erhitzte Metallplatte (Panel) getropft. Die Testdauer beträgt 20 Stunden, die Panel-Temperatur 260 °C und der Oelfluss 1 ml/Minute. Nach dem Test werden die Ablagerungen auf der Metallplatte, auf die das Oel tropft, durch Wiegen bestimmt. Eine zweite Bewertung erfolgt visuell. Bei der visuellen Beurteilung entsprechen die kleineren Werte den besten Ergebnissen. Als Schmieröl wird ein handelsübliches CD-Oel, welches mit dem Grundöl STANCO 150 verdünnt wird, verwendet. Diesem Oel werden die Stabilisatoren in einer Menge von 0,6 Gew.-%, bezogen auf das Oel, zugemischt und der DOPT-Prüfung unterzogen.
Die Ergebnisse sind in Tabelle 7 wiedergegeben.

**Tabelle 7**

| Stabilisator aus Beispiel Nr. | Ablagerungen auf Panel | |
|---|---|---|
| | Gewicht [mg] | visuell |
| keiner | 72,0 | 14 |
| 1 | 22,5 | 6 |

## Patentansprüche

1. Verbindungen der Formel I 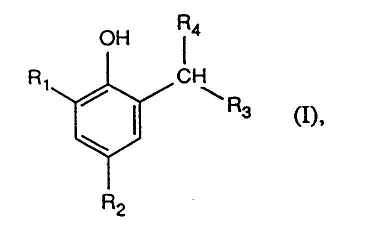 worin
R₁ C₁-C₄-n-Alkyl bedeutet,
R₂ C₄-C₁₈-tert-Alkyl oder α,α-Dimethylbenzyl ist,
R₃ für C₁-C₂₈-Alkyl steht und
R₄ Methyl oder Ethyl bedeutet,
mit der Massgabe, dass die Gruppe -CHR₃R₄ mindestens 4 C-Atome enthält.

2. Verbindungen der Formel I nach Anspruch 1, worin
R₁ Methyl oder Ethyl, insbesondere Methyl, bedeutet.

3. Verbindungen der Formel I nach Anspruch 1, worin
R₁ Methyl ist,
R₂ C₄-C₈-tert-Alkyl, insbesondere tert-Butyl, bedeutet und
R₃ C₁-C₂₂-n-Alkyl, insbesondere C₈-C₂₂-n-Alkyl, ist.

4. Verbindungen der Formel I nach Anspruch 1, worin
R₄ Methyl ist.

5. Mischungen von Verbindungen der Formel I, worin R₄ Methyl bedeutet, mit solchen, worin R₄ Ethyl bedeutet.

6. Mischungen nach Anspruch 5, worin das Mischungsverhältnis 99:1 bis 1:99, insbesondere 99:1 bis 90:10, beträgt.

7. Mischungen nach Anspruch 5, worin in den Verbindungen der Formel I mit R₄ = Methyl die Kettenlänge von R₃ um eins grösser ist als in den Verbindungen der Formel I mit R₄ = Ethyl.

8. Zusammensetzung enthaltend (a) ein gegen thermischen, oxidativen oder aktinischen Abbau empfindliches organisches Material und (b) mindestens eine Verbindung der Formel I, eine Mischung gemäss Anspruch 5.

9. Zusammensetzung nach Anspruch 8, worin (a) ein Polystyrol, ein substituiertes Polystyrol, ein Co- oder Terpolymeres von Styrol oder substituiertem Styrol, ein Polycarbonat, ein Polyestercarbonat, ein Polyurethan, ein Polyamid, ein Copolyamid, ein Polyacetal, ein Polyphenylenoxid, ein Polyolefin, ein Mineralöl, ein pflanzliches oder tierisches Oel oder Fett, ist.

10. Zusammensetzung nach Anspruch 8, worin die Komponente (a) ein Polystyrol, substituiertes Polystyrol oder Co- oder Terpolymeres von Styrol oder substituiertem Styrol, insbesondere schlagfestes Polystyrol (IPS), ein Styrol-Acrylnitril-Copolymer (SAN), ein Acrylnitril-Butadien-Styrol-Terpolymer (ABS) oder ein Methacrylnitril-Butadien-Styrol-Terpolymer (MBS), ist.

11. Zusammensetzung nach Anspruch 8, worin die Komponente (a) ein Polycarbonat, Polyestercarbonat, Polyurethan, Polyamid, Copolyamid, Polyacetal oder Polyphenylenoxid ist.

12. Zusammensetzung nach Anspruch 8, worin die Komponente (a) ein Polyolefin ist.

13. Verwendung von Verbindungen der Formel I, bzw. Mischungen gemäss Anspruch 5 zum Stabilisieren von gegen thermischen, oxidativen oder aktinischen Abbau empfindlichem organischem Material.

## Claims

1. A compound of formula I 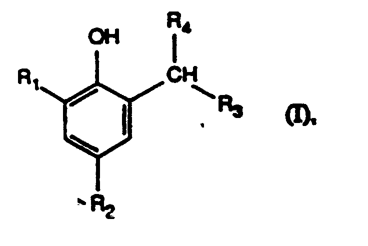 wherein
R₁ is C₁-C₄n-alkyl,
R₂ is C₄-C₁₈-tert-alkyl or α,α-dimethylbenzyl,
R₃ is C₁-C₂₈alkyl, and
R₄ is methyl or ethyl,
with the proviso that the group -CHR₃R₄ contains at least 4 carbon atoms,

2. A compound of formula I according to claim 1, wherein R₁ is methyl or ethyl, especially methyl.

3. A compound of formula I according to claim 1, wherein R₁ is methyl, R₂ is C₄-C₈tert-alkyl, especially tert-butyl, and R₃ is C₁-C₂₂n-alkyl, especially C₈-C₂₂n-alkyl.

4. A compound of formula I according to claim 1, wherein R₄ is methyl.

5. A mixture of compounds of formula I wherein R₄ is methyl with those wherein R₄ is ethyl.

6. A mixture according to claim 5, wherein the mixing ratio is from 99:1 to 1:99, especially from 99:1 to 90:10.

7. A mixture according to claim 5, wherein the chain length of R₃ in the compounds of formula I where R₄ = methyl is greater by one than in the compounds of formula I where R₄ = ethyl.

8. A composition comprising (a) an organic material which is susceptible to degradation induced by heat, oxidation or actinic light, and (b) at least one compound of formula I or one mixture according to claim 5.

9. A composition according to claim 8, wherein (a) is a polystyrene, a substituted polystyrene, a co- or terpolymer of styrene or substituted styrene, a polycarbonate, a polyester carbonate, a polyurethane, a polyamide, a copolyamide, a polyacetal, a polyphenylene oxide, a polyolefin, a mineral oil, a vegetable or animal oil or fat.

10. A composition according to claim 8, wherein component (a) is a polystyrene, substituted polystyrene or co- or terpolymer of styrene or substituted styrene, especially impact-resistant polystyrene (IPS), a styrene-acrylonitrile copolymer (SAN), an acrylonitrile-butadiene-styrene terpolymer (ABS) or a methacrylonitrile-butadiene-styrene terpolymer (MBS).

11. A composition according to claim 8, wherein component (a) is a polycarbonate, polyester carbonate, polyurethane, polyamide, copolyamide, polyacetal or polyphenylene oxide.

12. A composition according to claim 8, wherein component (a) is a polyolefin.

13. The use of a compound of formula I or of a mixture according to claim 5, for stabilising organic material which is susceptible to degradation induced by heat, oxidation or actinic light.

## Revendications

1. Composés de formule I 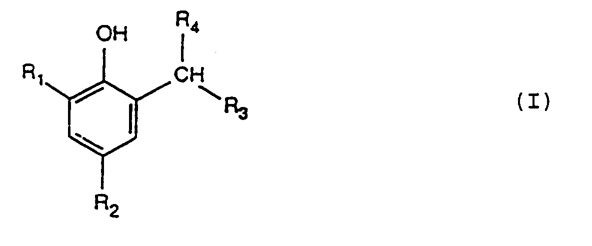 où
R₁ représente un groupe alkyle en C₁-C₄,
R₂ représente un groupe tert-alkyle en C₄-C₁₈ ou l'α,α-diméthylbenzyle
R₃ représente un groupe alkyle en C₁-C₂₈, et
R₄ un groupe méthyle ou éthyle,
à la condition que le groupe -CHR₃R₄ comporte au moins 4 atomes de carbone.

2. Composés de formule I selon la revendication 1, où
R₁ représente un groupe méthyle ou éthyle, notamment méthyle.

3. Composés de formule I selon la revendication 1, où
R₁ représente un groupe méthyle,
R₂ représente un groupe tert-alkyle en C₄-C₈, en particulier tert-butyle, et
R₃ représente un groupe n-alkyle en C₁-C₂₂, en particulier n-alkyle en C₈-C₂₂.

4. Composés de formule I selon la revendication 1, où
R₄ est un groupe méthyle.

5. Mélanges de composés de formule I, dans lesquels R₄ représente un groupe méthyle, avec ceux dans lesquels R₄ représente un groupe éthyle.

6. Mélanges selon la revendication 5, dans lesquels le rapport d'ingrédients est de 99:1 à 1:99, notamment de 99:1 à 90:10.

7. Mélanges selon la revendication 5, où dans les composés de formule I avec R₄ = méthyle, la longueur de chaîne de R₃ est plus grande de un que dans les composés de formule I avec R₄ = éthyle.

8. Composition contenant (a) une matière organique sensible à la dégradation thermique, oxydative ou actinique et (b) au moins un composé de formule I, un mélange selon la revendication 5.

9. Composition selon la revendication 8, où (a) est un polystyrène, un polystyrène substitué, un co- ou terpolymère de styrène ou de styrène substitué, un polycarbonate, un polyestercarbonate, un polyuréthanne, un polyamide, un copolyamide, un polyacétal, un poly(oxyphénylène), une polyoléfine, une huile minérale, une huile végétale ou animale ou une graisse.

10. Composition selon la revendication 8, dans laquelle le composant (a) est un polystyrène, un polystyrène substitué ou des co- ou terpolymères de styrène ou de styrène substitué, notamment le polystyrène résistant au choc (IPS), un copolymère styrène-acrylonitrile (SAN), un terpolymère acrylonitrile-butadiène-styrène (ABS) ou un terpolymère méthacrylonitrile-butadiène-styrène.

11. Composition selon la revendication 8, dans laquelle le composant (a) est un polycarbonate, un polyestercarbonate, un polyurétahnne, un polyamide, un copolyamide, un polyacétal ou un poly(oxyphénylène).

12. Composition selon la revendication 8, dans laquelle le composant (a) est une polyoléfine.

13. Utilisation de composés de formule I, respectivement de leurs mélanges selon la revendication 5, pour la stabilisation de matière organique sensible à la dégradation thermique, oxydative ou actinique.
